(19) Europäisches Patentamt
European Patent Office
Office européen des brevets

(11) EP 1 003 557 B1

(12) **EUROPEAN PATENT SPECIFICATION**

(45) Date of publication and mention of the grant of the patent:
**02.05.2003 Bulletin 2003/18**

(21) Application number: **98936573.9**

(22) Date of filing: **22.07.1998**

(51) Int Cl.[7]: **A61K 47/48**

(86) International application number:
**PCT/HU98/00066**

(87) International publication number:
**WO 99/004822 (04.02.1999 Gazette 1999/05)**

(54) **NEW SALTS WITH BENEFICIAL ORGANOLEPTIC PROPERTIES**

NEUE SALZE MIT NÜTZLICHEN ORGANOLEPTISCHEN EIGENSCHAFTEN

SELS A PROPRIETES ORGANOLEPTIQUES BENEFIQUES

(84) Designated Contracting States:
**GB**
Designated Extension States:
**AL LT LV MK RO SI**

(30) Priority: **25.07.1997 HU 9701293**

(43) Date of publication of application:
**31.05.2000 Bulletin 2000/22**

(73) Proprietor: **CHINOIN**
**Gyogyszer és Vegyészeti Termékek Gyára RT.**
**H-1045 Budapest IV (HU)**

(72) Inventors:
- **LEDNICZKY, Lászlo**
**H-1082 Budapest (HU)**
- **SERES, István**
**H-1044 Budapest (HU)**

(74) Representative: **Schwarz, Albin, Dr. et al**
**Kopecky & Schwarz**
**Patentanwälte**
**Wipplingerstrasse 32/22**
**1010 Wien (AT)**

(56) References cited:
**BE-A- 621 917**
**GB-A- 2 222 772**
**US-A- 4 176 180**
**US-A- 5 102 665**

- **MARYANOFF, B. E. ET AL.: "Structure-Activity Studies on Antidepresant 2,2-Diarylethylamines" J. MED. CHEM., vol. 27, 1984, pages 1067-1071, XP002083434**
- **ZELESKO, M. J. ET AL: "Cardiac-Slowing Amidines Containing the 3-Thioindole Group. Potential Antianginal Agents." J. MED. CHEM., vol. 26, 1983, pages 230-237, XP002083435**
- **MARYANOFF, B. E. ET AL.: "Stereochemistry in a Medium Sized Ring. Highly Diasteroselective N-Oxidation of a Substituted 3-benzazonine. X-Ray Crystal Structure of an Unusual Complex between as Amine N-Oxide and Saccharin" J. ORG. CHEM, vol. 55, 1990, pages 760-764, XP002083436**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 003 557 B1

## Description

The subject of the invention are compounds of the general formula (I) and their solvates that have organoleptically beneficial properties, pharmaceutical preparations containing the above mentioned compounds and/or their solvates, as well as a preparation of compounds of the general formula (I).

The disagreeable, bitter taste occurring during or right after oral administration is typical to the wide range of drug substances. These include various active ingredients such as drotaverin, prenoxdiazine, azithromycin, cimetidine, ciprofloxacin, erythromycin, fluoxetine, clarithromycin and ranitidine.

The bitter taste of a drug substance that is made up in form of fluid suspension can be observed during drinking the suspension or right after swallowing it. Therewith the bitter taste of a preparation that contains a bitter active ingredient can be sensed during the administration only if this bitter agent - due to keeping it in mouth for too long, chewing it accidentally or releasing in any other way - gets into contact with gustatory (taste) buds.

Generally, among routes of administration for several drug substances the oral dosage form is favored because it allows a simple and cheap dosage. However, it should often be considered, how cooperating patients are when they must take a tablet, capsule or suspension. Patients have numerous reasons why they do not want or are not able to ingest preparations administered orally, including, for example, the repugnant appearance of a dose form, its too large size, bad taste, or simply the fear that the unchewed drug can be stuck in the throat. Patient who have difficulty in taking oral dosage forms often retch, which really prevents oral administration. This problem is very usual among kids, but happens to adults as well.

Therefore it is desirable to formulate pharmaceutics in such a way by which the above mentioned problems can be eliminated. Generally coating and masking are useful to conceal the bitterness of drug substances. Accordingly, for example, chewable tablets had been developed, and they were increasingly accepted by patients, both children and others, having troubles with swallowing tablets or capsules as a whole. However, it happens very often that the drug substance has such a bitter taste that it is really unbearable to chew it, and the bad taste or after-taste caused by that bitter ingredient prevents patients from accepting oral administration.

<US-A - 4,176,180 describes suspendable pharmaceutical compositions containing erythromycine base and metoclopramide hydrochloride as active ingredients. As a usual excipient, among others, saccharinate is used.

Therefore we aimed to find derivatives of drug substances that would not represent the bad taste or after-taste of drug substances, but therapeutically are still equal to the known forms of pharmaceutical active ingredients, mostly hydrochloride salts.

We can surprisingly achieve our aim not by coating or masking but by producing salts or salt-solvates of drug substances formed with a sweetener, and then use them as active ingredients, or as a proportion of active ingredients, for pharmaceutical preparations.

We use the term of drug substance for the active ingredients of any human or animal drugs, whose organoleptic properties, especially taste and after-taste, are unpleasant for patients or certain groups of patients or for animals.

Sweeteners are defined here as every natural or synthetic sweetener that is used to sweeten human food or animal feed, and is known from literature at the time of filing of this patent application. Useful sweeteners are, for example, saccharin, acesulpham, aspartame, alitam, cyclohexylamino-sulphonic acid, glycirrizine acid and similar compounds.

Although we use the term of drug substance mainly for the above listed compounds, we do not exclude any other compounds that have unpleasant organoleptic properties.

The drug substances from which the new derivatives of the general formula (I) can be prepared include, but are not limited to the following: drotaverin, prenoxdiazine, selegiline, drug substances with phenthiazin structure, ciprofloxacin, fluoxetine, enalapril, clopidogrel, irbesartan, azithromycin, erythromycin, clarithromycin, cimetidine and ranitidine.

The new compounds of the general formula (I) show the same qualitative therapeutical profile as the starting known pharmaceutically active ingredients and its known salts. For example as it is known the spasmolytic drotaverin and its hydrochloride are inhibitors of phosphodiesterase IV isoenzime (PDE-IV) (EP-0664127A2). The new drotaverin salts and their solvates represented by the general formula (I) are selective PDE-IV inhibitors as well:

| **Compound** | **Human U937 (cell line) PDE-IV enzime inhibition $IC_{50}$** |
|---|---|
| drotaverin hydrochloride | $4.7 \cdot 10^{-6}$ mol |
| drotaverin cyclamate (Example 2) | $1 \cdot 10^{-6}$ mol |
| drotaverin saccharimide (Example 1) | $3.8 \cdot 10^{-6}$ mol |

**ASSAY:**

Cyclic nucleotide phosphodiesterase activity is measured by a two-step procedure.
The standard assay mixture contains:

30μl of PDE IV enzyme,
20 μl of test compound or its solvent,
20μl of [$^3$H]cAMP substrate (220000 dpm)
30 μl of 40 mM Tris buffer (pH=7.6)
containing 2.5 mM $MgSO_4$.

The reaction is initiated by addition of the substrate.
Samples are incubated at 30 °C for 30 min. and the reaction is stopped by boiling the assay tubes for 2 min.
After cooling the tubes, 50μl of Crotalux atrox (0.5 mg/ml dest. water) is added and the samples are incubated and shaked for 30 min at 37°C.
The nonreacted cAMP is removed by chromatography on Dowex 1x8 (Cl-form): 1 ml of Dowex is added to the sample and after vortex-mixing the resin is left to settle for 60 min. at room temperature. The mixture of 500 μl supernatant and 5 ml of liquid scintillation cocktail is measured to determine the amount of formed $^3$H adenosine.

**RESULTS CALCULATION:**

$$\text{inhibition\%} = 100 - \frac{I-C}{T-C}$$

I : enzyme activity in the presence of inhibitor
C : control (0 time)
T : total enzyme activity
$IC_{50}$ values were calculated by using of Medusa Version 1.4.

Furthermore in Figures 1 to 7 it is demonstrated that the pharmacological profiles are the same in case of drotaverin salts of the general formula (I) and the known drotaverin hydrochloride.

**Figure 1**

The spontaneous tracheal tone relaxant effect of two drotaverine salts was tested.
Both salts concentration-dependently relaxed the tracheal tone. As it can be seen in Figure 1 there is no significant difference between the two salts in the respect of efficacy.

**Figure 2**

The histamine precontracted tracheal tone relaxant effect of the two drotaverine salts were tested. Both salts concentration-dependently relaxed the histamine induced tracheal contracture. As it can be seen in Figure 2 there is no significant difference between the two salts in the respect of efficacy.

**Figure 3**

The inhibitory effect of the two drotaverine salts was tested on allergen (ovalbumin) induced tracheal contraction on isolated tracheal preparation derived from ovalbumin presensitized guinea pigs. The area under the ovalbumin induced contraction curve was calculated (AUC) and its percentage decrease was used for the characterisation of drug effect. Both salts concentration-dependently decreased the allergen response. There was not significant difference between the two salts in this respect.

**Figure 4**

The in vivo broncholitic effect of the two drotaverine salts were tested on allergen (ovalbumin) induced bronchoconstriction in allergen (ovalbumin) presensitized guinea pigs after intraduodenal administration. Both drotaverine

salts shows dose-dependent broncholitic effect. There is no significant difference between the two salts in the respect of broncolitic effect.

**Figure 5**

The time duration of the broncholitic effect of the equimolar dose (~$ED_{50}$ dose) of the two drotaverine salts were tested on allergen (ovalbumin) induced bronchoconstriction test. Both salts have long-lasting broncholitic effect. The difference between the two time curves is not relevant so the pharmacological effect of the two salts are practically identical.

**Figure 6**

The in vivo broncholitic effect of the two drotaverine salts were tested on histamine induced bronchoconstriction test in guinea pigs after intraduodenal administration.

Both drotaverine salts shows dose-dependent broncholitic effect. There is no significant difference between the two salts in the respect of broncolitic effect.

**Figure 7**

The allergen (ovalbumin) presensitized animals were treated by the equimolar doses of drotaverine salts for seven days (single p.o. dose/day). On the 8th day half of the animals of each salt treated group were treated by vehicle and 30 min. later the bronchoconstriction was evoked by the i.v. injection of allergen (ovalbumin). Both salts have significant broncholitic effect even 24 hours after the last treatment.

There is no significant difference between the two salts in the respect of efficacy.

The other half of each salt treated group was treated again by the adequate salt and 30 min. later the bronchoconstriction was evoked by the i.v. injection of allergen.

The acute broncholitic effect following subchronic treatment of both salts was higher as was expected on the basis of acute tests (see Figure 4). There is no significant difference between the two salts in the respect of efficacy.

Consequently the above new drotaverin salts and their solvates showing pleasant sweet taste are of great use in pharmaceuticals such as spasmolytic agents, antidepressive agents, tranquilizing agents, antidemential agents, antiinflammatory agents, antiallergic agents, antiasthmatic agents, liver-protecting agents, diuretic agents, etc., and also in medicaments for the prevention and therapy of various diseases including distortions of the central nervous system such as depression and dementia as well as others such as inflammation, allergic diseases, asthma, liver diseases and kidney diseases.

Compounds of the general formula (I) can be prepared by the reaction of the positive charged form (cation) of a drug substance with the negative charged form (anion) of a sweetener, using methods known per se. The above reaction is advantageously carried out in a solvent, and the compounds with general formula (I) are obtained and purified using common techniques.

The used drug substances and the sweeteners are commercial products.

Pharmaceutical preparations can be produced from compounds of the general formula (I) by common formulation techniques and using accepted fillers, diluents, lubricants, binding agents, pigments and stabilizing agents.

It should be noted that the compounds of the general formula (I) can show unobvious advantages in the field of bioavailability and/or formulation technology. We describe further details for our invention by the following examples, without limiting our claims to these examples.

**Example 1**

A 40°C solution of 20 g drotaverin base in 20 $cm^3$ absolute ethanol was added to the solution of 9.02 g saccharimide 60 $cm^3$ hot, absolute ( anhydrous) ethanol. The solution undergoing crystallization was cooled slowly; when it reached 10°C, it was clarified and covered with alcohol; the obtained 28.2 g of crude drotaverin-saccharimide ethanolate was recrystallized from 96% alcohol of threefold volume, with a yield of 95%. The obtained crystals are light yellow, MP: 95-97°C.

It is proven by the data of elementary analyses and spectra (IR, NMR) that the resulting substance is the solvate - containing one mole ethanol - of the salt formed by the reaction of drotaverin and saccharimide.

The elementary analysis data calculated for the empirical formula of $C_{31}H_{42}N_2O_8S.C_2H_5OH$ are: (calculated C%=63.24; H%=6.75, N%=4.47, S%=5.12; measured C%=63.59, H%=6.68, N%=4.37, S%=5.25)

Assignation:

**IR** (Bruker IFS28 KBr pellet) 3498 vOH ethanol; 3085, 3064 vCH(aromatic); 2978, 2933, 2882 vCH (aliphatic); 2788-2338VNH$^+$; 1654 vC=O; 1610, 1571, 1518 aromatic structure vibration; 1258, 1044 vC-O-C
$^1$**H NMR** (Bruker DRX-400; 400 Mhz $CDCl_3$) 7.79 m [2H] saccharin 4.7-H; 7.58 m [2H] saccharin 5.6-H 7.28 s [1H] 8-H, 6.95 d [1H] 2'-H $J_{2',6}$=1.9; 6.82 dd [1H] 6'-H $J_{5',6}$=8.2; 6.75 d 6'-H; 6.73 s [1H] 5-H; 4.49 s [2H] $CH_2$; 4.17 q [2H] 6-$OCH_2$ $J_{OCH2.CH3}$=7.0; 4.09 m [2H] 3-$H_2$; 4.00 q [6H] 7-$OCH_2$, 3'-$OCH_2$, 4'-$OCH_2$; 3.72 q [2H] ethanol $OCH_2$; 3.00 m [2H] 4-H2; 1.48 t [3H], 1.40 t [3H], 1.38 t[3H], 1.33 t [3H] $CH_3$; 1.22 t [3H] ethanol $CH_3$.

**Example 2**

21.6 g drotaverin-hydrochloride was dissolved in 40 $cm^3$ of 96%, hot alcohol and then a solution of 10.2 g sodium cyclamate in 25 $cm^3$ distilled water was added. By cooling the solution, drotaverin cyclamate salt was crystallized at 32°C; after further cooling, it was kept at 5°C to crystallize for two hours, and then clarified, covered and dried with 50% alcohol;
Weight: 14.4 g (Color: almost white)
The crude product was recrystallized from 96% alcohol of threefold volume, with a yield of 80%; the obtained product is almost white. MP: Melting begins at 158 ° C and it is a long lasting process.
It is proven by the data of elementary analyses and spectra (IR, NMR) that the resulting substance is the salt formed by the reaction of drotaverin and cyclohexylamino-sulphonic acid.

Assignation:

**IR** (Bruker IFS28, KBr pellet) 3285 vNH; 3093, 3056 vCH (aromatic); 2975, 2923, 2854 νCH (aliphatic); 1661νC≡N; 1603, 1562, 1518 aromatic structure vibration, 1278 1035 vC-O-C
$^1$**H NMR** (Bruker DRX-400; 400 Mhz $CDCl_3$) 7.24 s [1H] 8-H, 6.94 d [1H] 2'-H $J_{2',6'}$= 1.9; 6.82 dd [1H] 6'-H $J_{5',6'}$=8.2; 6.77 d 6'-H;6.73 s [1H] 5-H; 4.36 s [2H] $CH_2$; 4.17 q [2H] 6-$OCH_2$ $J_{OCH2.CH3}$=7.0; 4.09 q [2H] 7-$OCH_2$ $J_{OCH2.CH3}$=7.0; 4.02 m [2H] 3-$H_2$; 4.03 q [2H], 3.99 q [2H], 3'-$OCH_2$, 4'-$OCH_2$ $J_{OCH2.CH3}$=7.0; 3.28 m [1H] cyclamate 1-H; 2.98 m [2H] 4-$H_2$; 1.49 t [3H], 1.42 t [3H], 1.41 t [3H], 1.40 t [3H] $CH_3$; 2.11 m [2-H] 1.68 m [2H] 1.55 m [1H], 1.4-1.05 m [5H] cyclamate 2,3,4,5,6-$H_2$

If the product was crystallized from water-organic solvent mixture, a product containing 1 mole of water as hydrate can be separated. In case of this monohydrate of drotaverin cyclamate the $^1$HNMR spectrum is the same as in case of the water free salt but the IR spectrum is the following:

**IR** (Bruker IFS28, KBr pellet) 3285 vNH; 3093, 3056 vCH (aromatic); 2975, 2923, 2854 νCH (aliphatic); 1646 vC ≡ N; 1603, 1562, 1518 aromatic structure vibration, 1278, 1035 vC-O-C; νOH($H_2O$) 3477, 3441.

**Example 3**

10.9 g prenoxdiazine-base and 5.55 g (each 0.03 mole) saccharimide was dissolved in 75 $cm^3$ ebullient anhydrous ethanol. The clear solution was subject to crystallization by cooling and mixing; the resultant crystals were drawn off and covered with some ethyl alcohol. The crude product was subject to vacuum drying.
Weight: 16.03 g (97.4%) MP: 131-132°C.
The obtained prenoxdiazine saccharimide salt was subject to crystallization using anhydrous alcohol of 5.2-fold volume with a yield of 96%; MP: 131-132°C.

Elementary analyses of $C_{30}H_{32}N_4O_4S$:

| C% | H% | N% | S% |
|---|---|---|---|
| 66.16 | 5.92 | 10.29 | 5.89 |

(continued)

| C% | H% | N% | S% |
|---|---|---|---|
| 66.45 | 6.06 | 10.48 5.92 | |

It is proven by the data of elementary analyses and spectra (IR, NMR) that the resulting substance is the salt obtained by the reaction of prenoxdiazine and saccharimide.

Assignation:

**IR** (Bruker IFS28 KBr pellet) 3064, 3035, 3013; νCH (aromatic); 2962, 2940, 2919, 2868 νCH (aliphatic); 2766-2120 $νNH^+$; 1644 saccharin νC=O: 1583, 1525, 1495; aromatic structure vibration; 751, 716 monosubs. aromatic
**$^1$H NMR** (Bruker DRX-400, 400 MHz, $CDCl_3$), 7.79 m [2H] saccharin 4.7-H; 7.62 m [2H] saccharin 5.6-H; 7.3-7.15 m [10H] aromatic-H; 4.60 t [1H] CH, $J_{CH,CH2}$=8.2; 3.55 t [2H] $NCH_2$ $J_{NCH2,5\text{-}CH2}$=7.4; 3.44 m [4H] 3-$CH_2$ 5-$CH_2$; 3.1 b m [4H] 2'6'-$H_2$;1.98 b [4H];1.65 b [2H] 3',4',5'-$H_2$

**Example** 4

<u>Dry syrup</u>

The following components were mixed:

| | |
|---|---|
| drotaverin cyclamate | 10.0 g (Example 2) |
| sodium pyrosulfit | 3.0 g |
| citric acid | 3.0 g |
| Keltrol ® | 5.0 g |
| Nipagin ® M | 5.0 g |
| saccharose | 10.0 g |
| mannit | 5.0 g |
| flavoring material | qu. sat. |

This mixture mixed with drinking water ad 100 ml gave the liquid syrup form with total volume of 100 ml.
Examination of organoleptical properties of above composition showed that the characteristic bitter taste of drotaverin did not appear.

**Example 5**

Ready to use suspension

The same components were mixed as in Example 4 but aqua dest. was added to the solid mixture and it was bottled.
Examination of organoleptical properties of above composition showed that the characteristic bitter taste of drotaverin did not appear.

**Example 6**

| Tablet composition | |
|---|---|
| 150 g | drotaverin saccharimide (Example 1) |
| 5 g | magnesium stearate |
| 8 g | talcum |
| 50 g | pregelatinized starch |

(continued)

| Tablet composition | |
|---|---|
| 10 | g PVP |
| 100 g | lactose |
| 90 g | microcrystalline cellulose |

were mixed, granulated and compressed into tablets with total weight of 413 mg containing 150 mg of active substance.

Examination of organoleptical properties of above composition showed that the characteristic bitter taste of drotaverin did not appear.

## Claims

**1.** Compounds of the general formula (I)

$$A — X \qquad (I)$$

where

A is a positive charged ion (cation) of a drug substance,
X is a negative charged ion (anion) of sweetener - and their solvates having organoleptically beneficial properties.

**2.** Compounds of the general formula (I) and their solvates according to claim 1 where

A is as defined in claim
X means compounds containing negative charged ion of the formula (1):

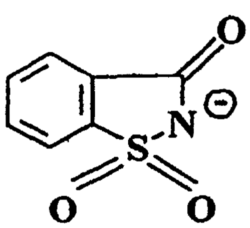

(1)

formula (2):

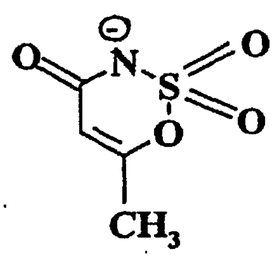

(2)

formula (3):

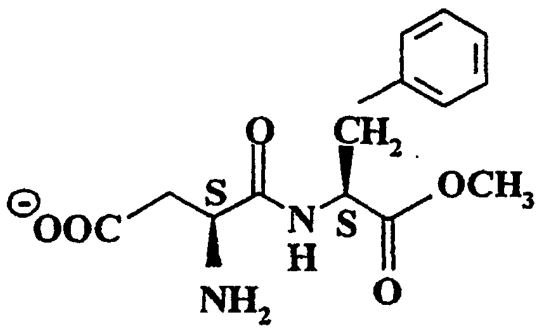

(3)

formula (4):

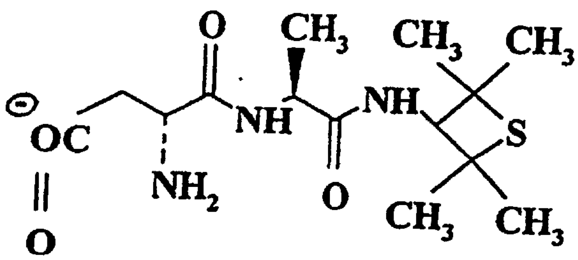

(4)

formula (5):

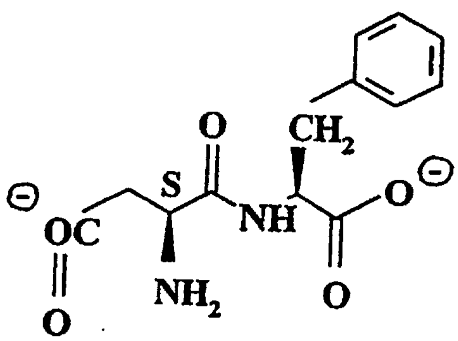

(5)

or formula (6) :

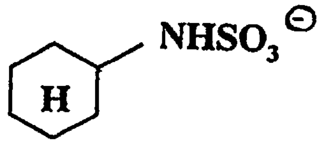

(6)

and their solvates.

**3.** Compounds of the general formula (I) and their solvates according to claim 1 - where

A is a positive charged ion of drotaverin, prenoxdiazine, selegiline, azithromycin, cimetidine, ciprofloxacin, clopidogrel, irbesartan, erythromycin, fluoxetine, ranitidine, clarithromycin, enalapril.

**4.** Salt of drotaverin formed by the anion of the formula (1) and its solvates.

**5.** Salt of drotaverin formed by the anion of the formula (2) and its solvates.

**6.** Salt of drotaverin formed by the anion of the formula (3) and its solvates.

**7.** Salt of drotaverin formed by the anion of the formula (4) and its solvates.

**8.** Salt of drotaverin formed by the anion of the formula (5) and its solvates.

**9.** Salt of drotaverin formed by the anion of the formula (6) and its solvates.

**10.** Salt of prenoxdiazine formed by the anion of the formula (1) and its solvates.

**11.** Salt of prenoxdiazine formed by the anion of the formula (2) and its solvates.

**12.** Salt of prenoxdiazine formed by the anion of the formula (3) and its solvates.

**13.** Salt of prenoxdiazine formed by the anion of the formula (4) and its solvates.

**14.** Salt of prenoxdiazine formed by the anion of the formula (5) and its solvates.

**15.** Salt of prenoxdiazine formed by the anion of the formula (6) and its solvates.

**16.** Pharmaceutical preparations **characterized in that**, they contain at least one compound of the general formula (I), - where A and X are as defined in claim I - and/or its/their solvates.

**17.** Solid pharmaceutical preparations according to claim 16, **characterized in that**, they contain at least one compound of the general formula (I), - where A and X are as defined in claim 1 - and/or its/their solvates.

**18.** Liquid pharmaceutical preparations according to claim 16, **characterized in that**, they contain at least one compound of the general formula (I), - where A and X are as defined in claim 1 - and/or its/their sovates.

**19.** Process for the preparation of compounds of the general formula (I), **characterized in that**, a cation form (positive charged ion) of a drug substance is reacted with an anion form (negative charged ion) of a sweetener.

**20.** Procedure according to claim 19, **characterized in that**, the reaction is carried out in liquid medium.

**21.** An inhibitor for PDE-IV containing at least one of the compounds and/or their solvates according to claims 4 to 9 as therapeutically active component.

**22.** Use of at least one of the compounds and/or their solvates according to claims 4 to 9 for the preparation of pharmaceutical compositions for inhibiting PDE-IV enzime.

**23.** A spasmolytic agent containing at least one of the compounds and/or their solvates according to claims 4 to 9 as therapeutically active component.

**24.** Use of at least one of the compounds and/or their solvates according to claims 4 to 9 for the preparation of spasmolytic pharmaceutical compositions.

## Patentansprüche

**1.** Verbindungen der allgemeinen Formel (1)

$$A—X \qquad (I)$$

wobei

A ein positiv geladenes Ion (Kation) einer Arzneimittelsubstanz ist,
X ein negativ geladenes Ion (Anion) eines Süßungsmittels ist - und deren Solvate, welche organoleptisch günstige Eigenschaften haben.

**2.** Verbindungen der allgemeinen Formel (I) und deren Solvate gemäß Anspruch 1, wobei

A so ist wie im Anspruch definiert
X Verbindungen, die ein negativ geladenes Ion der Formel (1):

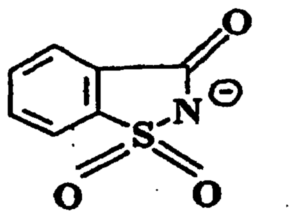

(1)

der Formel (2):

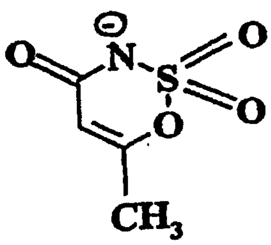

(2)

der Formel (3):

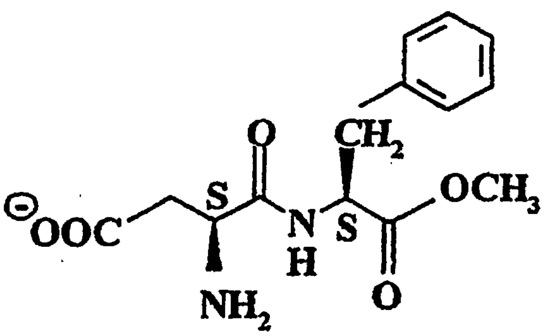

(3)

der Formel (4):

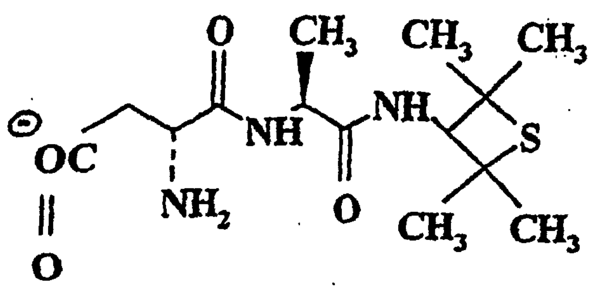

(4)

der Formel (5):

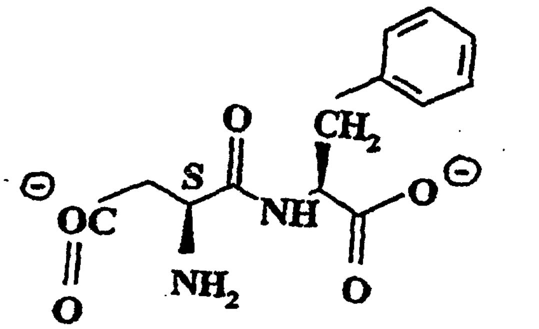

(5)

oder der Formel (6):

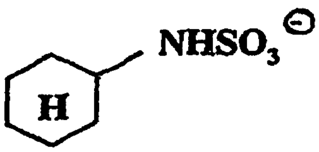

(6)

enthalten, sowie deren Solvate bedeutet.

**3.** Verbindungen der allgemeinen Formel (I) und deren Solvate gemäß Anspruch 1, wobei

A ein positiv geladenes Ion von Drotaverin, Prenoxdiazin, Selegilin, Azithromycin, Cimetidin, Ciprofloxacin, Clopidogrel, Irbesartan, Erythromycin, Fluoxetin, Ranitidin, Clarithromycin, Enalapril ist.

**4.** Salz von Drotaverin, gebildet durch das Anion der Formel (1), und dessen Solvate.

**5.** Salz von Drotaverin, gebildet durch das Anion der Formel (2), und dessen Solvate.

**6.** Salz von Drotaverin, gebildet durch das Anion der Formel (3), und dessen Solvate.

**7.** Salz von Drotaverin, gebildet durch das Anion der Formel (4), und dessen Solvate.

**8.** Salz von Drotaverin, gebildet durch das Anion der Formel (5), und dessen Solvate.

**9.** Salz von Drotaverin, gebildet durch das Anion der Formel (6), und dessen Solvate.

**10.** Salz von Prenoxdiazin, gebildet durch das Anion der Formel (1), und dessen Solvate.

**11.** Salz von Prenoxdiazin, gebildet durch das Anion der Formel (2), und dessen Solvate.

**12.** Salz von Prenoxdiazin, gebildet durch das Anion der Formel (3), und dessen Solvate.

**13.** Salz von Prenoxdiazin, gebildet durch das Anion der Formel (4), und dessen Solvate.

**14.** Salz von Prenoxdiazin, gebildet durch das Anion der Formel (5), und dessen Solvate.

**15.** Salz von Prenoxdiazin, gebildet durch das Anion der Formel (6), und dessen Solvate.

**16.** Pharmazeutische Präparate, **dadurch gekennzeichnet, dass** sie zumindest eine Verbindung der allgemeinen Formel (I) - wobei A und X so sind wie im Anspruch 1 definiert - und/oder dessen/deren Solvate enthalten.

**17.** Feste pharmazeutische Präparate gemäß Anspruch 16, **dadurch gekennzeichnet, dass** sie zumindest eine Verbindung der allgemeinen Formel (I) - wobei A und X so sind wie im Anspruch 1 definiert - und/oder dessen/deren

Solvate enthalten.

**18.** Flüssige pharmazeutische Präparate gemäß Anspruch 16, **dadurch gekennzeichnet, dass** sie zumindest eine Verbindung der allgemeinen Formel (I) - wobei A und X so sind wie im Anspruch 1 definiert - und/oder dessen/deren Solvate enthalten.

**19.** Verfahren zur Herstellung von Verbindungen der allgemeinen Formel (I), **dadurch gekennzeichnet, dass** eine Kationenform (ein positiv geladenes Ion) einer Arzneimittelsubstanz mit einer Anionenform (einem negativ geladenen Ion) eines Süßungsmittels umgesetzt wird.

**20.** Verfahren gemäß Anspruch 19, **dadurch gekennzeichnet, dass** die Reaktion in einem flüssigen Medium durchgeführt wird.

**21.** Hemmstoff für PDE-IV, enthaltend zumindest eine der Verbindungen und/oder deren Solvate gemäß den Ansprüchen 4 bis 9 als therapeutisch aktive Komponente.

**22.** Verwendung von zumindest einer der Verbindungen und/oder deren Solvaten gemäß den Ansprüchen 4 bis 9 zur Herstellung von pharmazeutischen Zusammensetzungen zur Hemmung des PDE-IV-Enzyms.

**23.** Krampflösendes Mittel, enthaltend zumindest eine der Verbindungen und/oder deren Solvate gemäß den Ansprüchen 4 bis 9 als therapeutisch aktive Komponente.

**24.** Verwendung von zumindest einer der Verbindungen und/oder deren Solvaten gemäß den Ansprüchen 4 bis 9 zur Herstellung von krampflösenden pharmazeutischen Zusammensetzungen.

## Revendications

**1.** Composés de formule générale (I)

$$A - X \qquad (I)$$

où A est un ion chargé positivement (cation) provenant d'une substance médicamenteuse,
X est un ion chargé négativement (anion) provenant d'un édulcorant, et leurs solvates ayant des propriétés organoleptiques bénéfiques.

**2.** Composés de formule générale (I) et leurs solvates selon la revendication 1, où
A est tel que défini dans la revendication,
X désigne des composés contenant un ion chargé négativement de formule (1) :

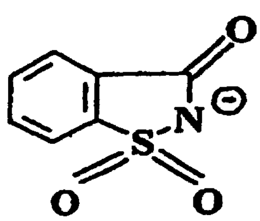

(1)

de formule (2) :

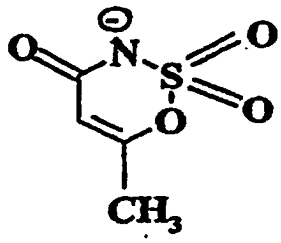

(2)

de formule (3) :

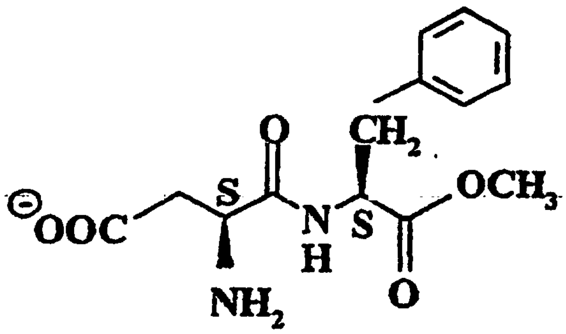

(3)

de formule (4) :

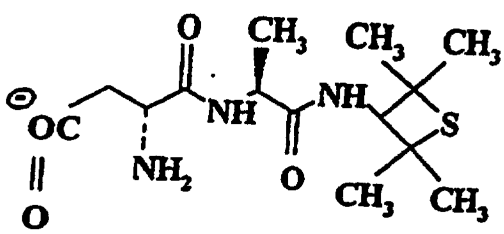

(4)

de formule (5) :

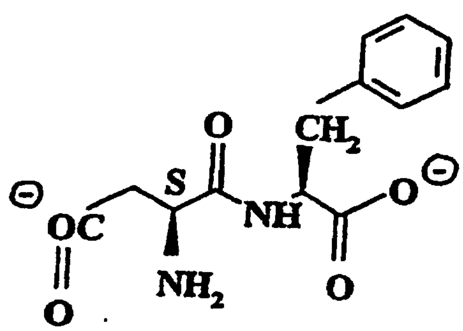

(5)

ou de formule (6) :

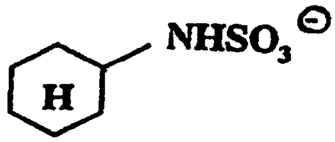

(6)

et leurs solvates.

3. Composés de formule générale (I) et leurs solvates selon la revendication 1, où A est un ion chargé positivement provenant de la drotavérine, de la prénoxdiazine, de la sélégiline, de l'azithromycine, de la cimétidine, de la ciprofloxacine, du clopidogrel, de l'irbésartan, de l'érythromycine, de la fluoxétine, de la ranitidine, de la clarithromycine, de l'énalapril.

4. Sel de la drotavérine formé par l'anion de la formule (1) et ses solvates.

5. Sel de la drotavérine formé par l'anion de la formule (2) et ses solvates.

6. Sel de la drotavérine formé par l'anion de la formule (3) et ses solvates.

7. Sel de la drotavérine formé par l'anion de la formule (4) et ses solvates.

8. Sel de la drotavérine formé par l'anion de la formule (5) et ses solvates.

9. Sel de la drotavérine formé par l'anion de la formule (6) et ses solvates.

10. Sel de la prénoxdiazine formé par l'anion de la formule (1) et ses solvates.

11. Sel de la prénoxdiazine formé par l'anion de la formule (2) et ses solvates.

12. Sel de la prénoxdiazine formé par l'anion de la formule (3) et ses solvates.

13. Sel de la prénoxdiazine formé par l'anion de la formule (4) et ses solvates.

14. Sel de la prénoxdiazine formé par l'anion de la formule (5) et ses solvates.

15. Sel de la prénoxdiazine formé par l'anion de la formule (6) et ses solvates.

16. Préparations pharmaceutiques, **caractérisées en ce qu'**elles contiennent au moins un composé de formule générale (I), où A et X sont tels que définis dans la revendication 1, et/ou son ou leurs solvates.

17. Préparations pharmaceutiques solides selon la revendication 16, **caractérisées en ce qu'**elles contiennent au moins un composé de formule générale (I), où A et X sont tels que définis dans la revendication 1, et/ou son ou leurs solvates.

18. Préparations pharmaceutiques liquides selon la revendication 16, **caractérisées en ce qu'**elles contiennent au moins un composé de formule générale (I), où A et X sont tels que définis dans la revendication 1, et/ou son ou leurs solvates.

19. Procédé pour la préparation de composés de formule générale (I), **caractérisé en ce que** l'on fait réagir une forme cationique (ion chargé positivement) d'une substance médicamenteuse avec une forme anionique (ion chargé négativement) d'un édulcorant.

20. Procédé selon la revendication 19, **caractérisé en ce que** la réaction est réalisée dans un milieu liquide.

21. Inhibiteur de la phosphodiestérase IV (PDE-IV) contenant au moins un des composés et/ou de leurs solvates selon les revendications 4 à 9 en tant que principe actif thérapeutique.

**22.** Utilisation d'au moins un des composés et/ou de leurs solvates selon les revendications 4 à 9 pour la préparation de compositions pharmaceutiques pour l'inhibition de l'enzyme PDE-IV.

**23.** Agent spasmolytique contenant au moins un des composés et/ou de leurs solvates selon les revendications 4 à 9 en tant que principe actif thérapeutique.

**24.** Utilisation d'au moins un des composés et/ou de leurs solvates selon les revendications 4 à 9 pour la préparation de compositions pharmaceutiques spasmolytiques.

A — X (I)

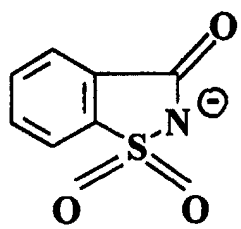

(1)

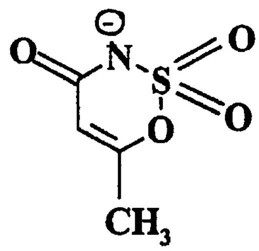

(2)

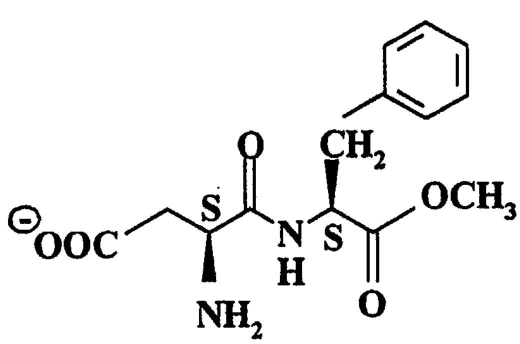

(3)

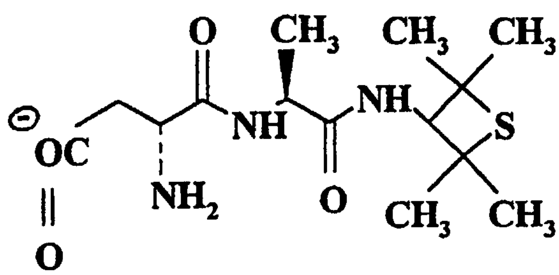

(4)

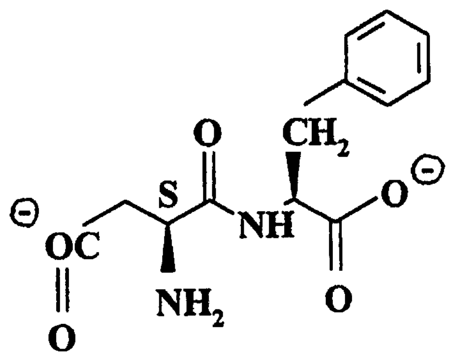

(5)

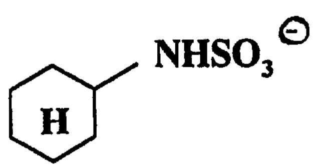

(6)

**Figure 1**

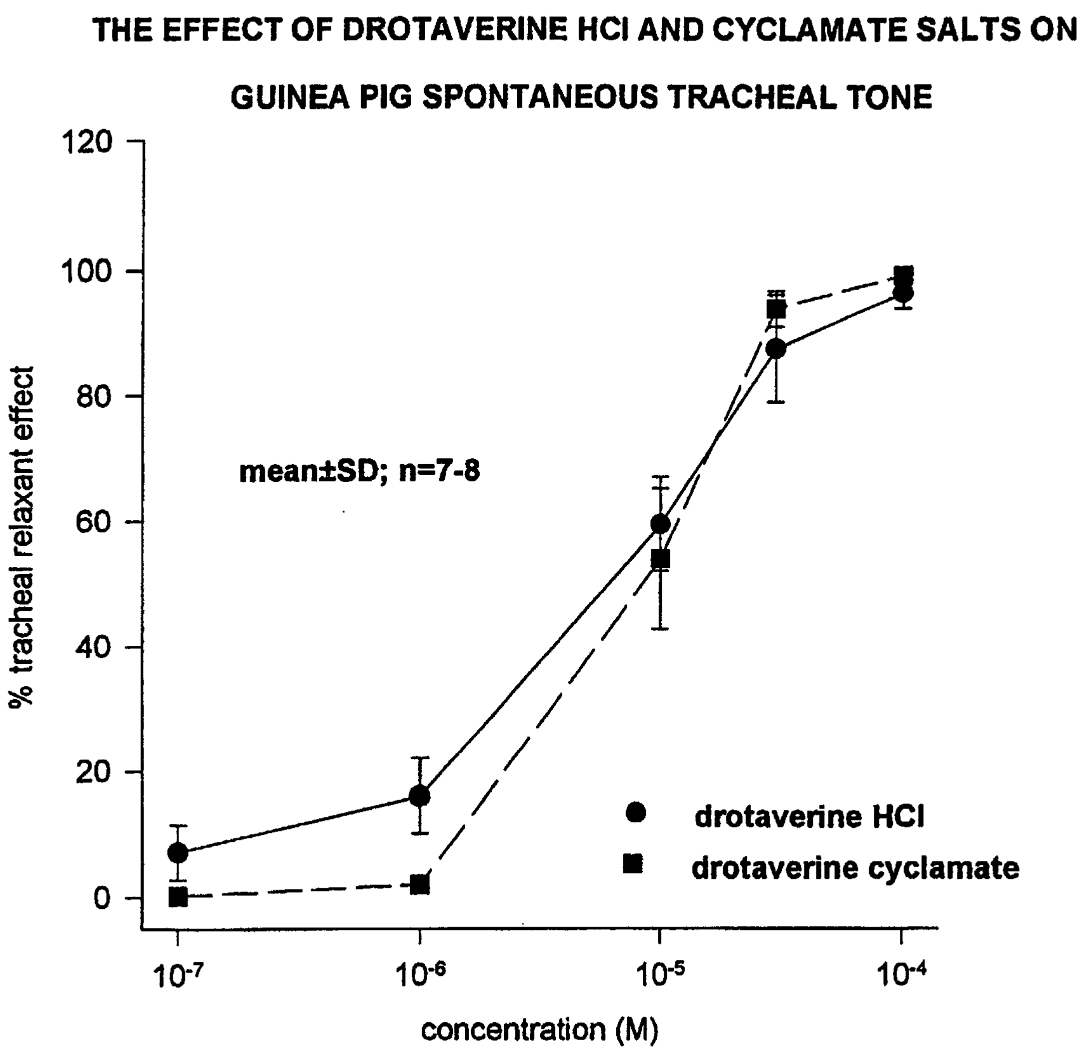
THE EFFECT OF DROTAVERINE HCl AND CYCLAMATE SALTS ON
GUINEA PIG SPONTANEOUS TRACHEAL TONE
120
100
80
60
40
20
0
% tracheal relaxant effect
mean±SD; n=7-8
drotaverine HCl
drotaverine cyclamate
10-7
10-6
10-5
10-4
concentration (M)

**Figure 2**

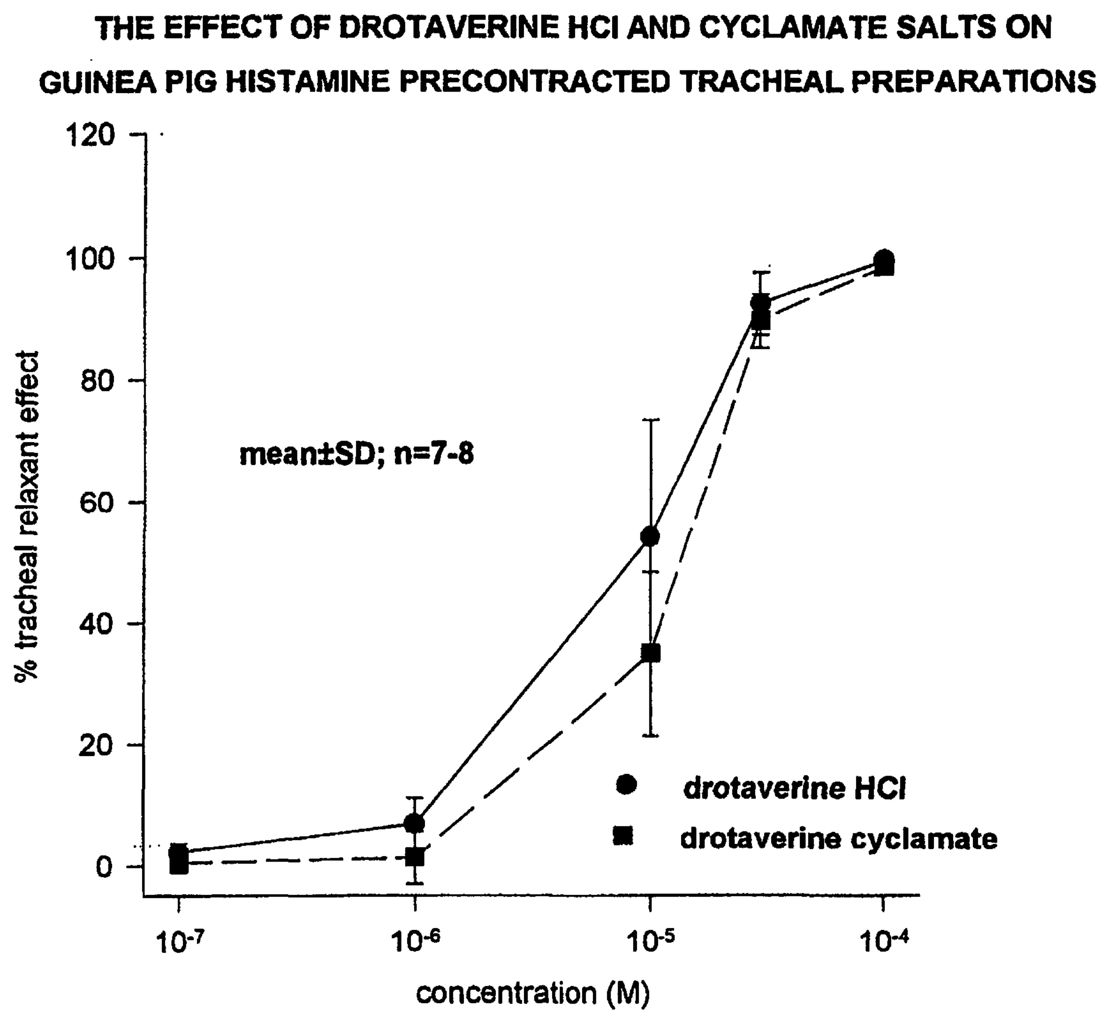
THE EFFECT OF DROTAVERINE HCl AND CYCLAMATE SALTS ON GUINEA PIG HISTAMINE PRECONTRACTED TRACHEAL PREPARATIONS
120
100
80
60
40
20
0
% tracheal relaxant effect
mean±SD; n=7-8
drotaverine HCl
drotaverine cyclamate
10-7
10-6
10-5
10-4
concentration (M)

**Figure 3**

**THE EFFECT OF DROTAVERINE CYCLAMATE AND HCl SALTS ON OVALBUMIN INDUCED GUINEA PIG TRACHEAL CONTRACTION**

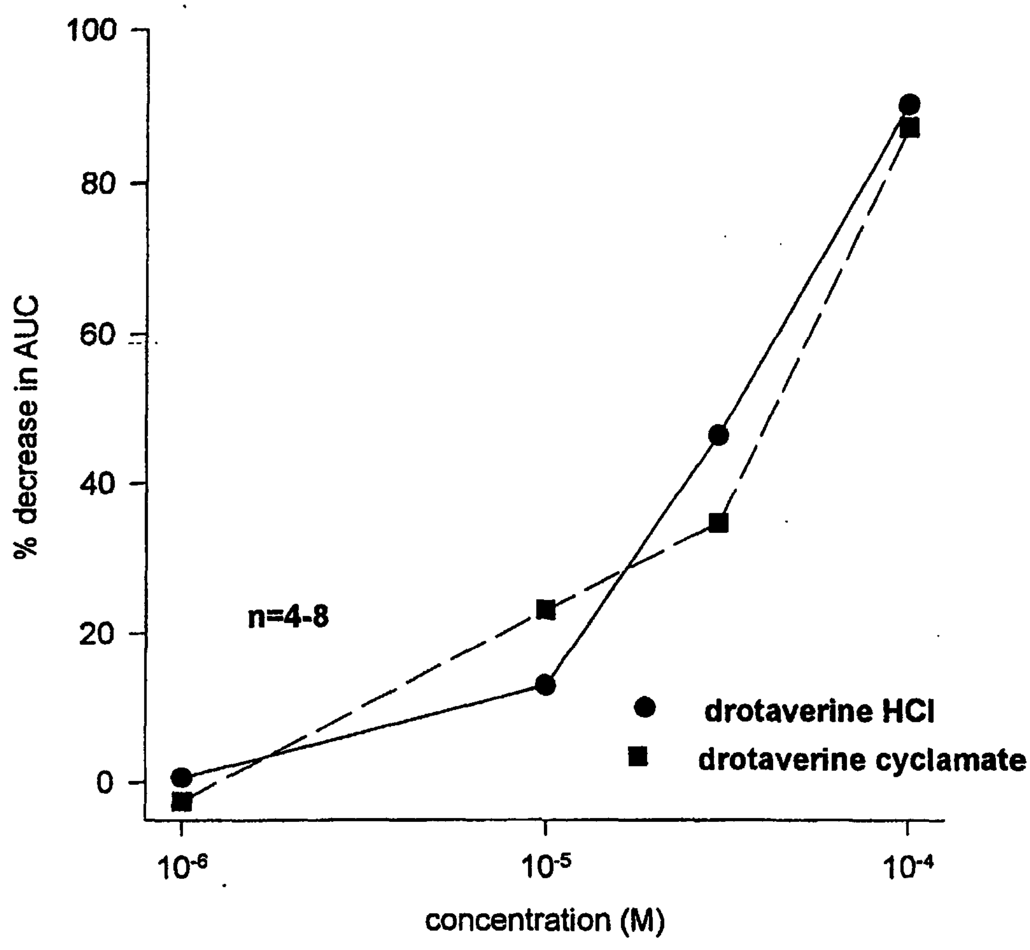

**Figure 4**

**THE EFFECT OF DROTAVERINE CYCLAMATE AND HCL SALTS ON ALLERGEN INDUCED BRONCHOCONSTRICTION IN ANAESTHETISED GUINEA PIGS 30 MIN AFTER INTRADUODENAL ADMINISTRATION**

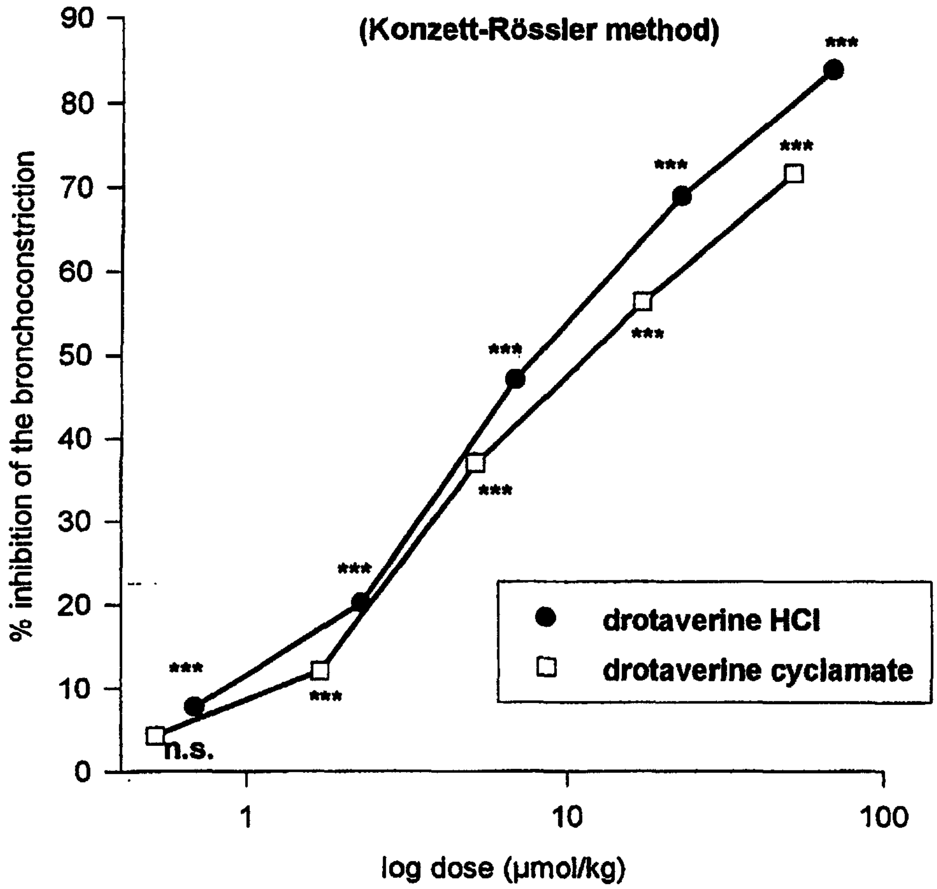


**Statistical analysis by Student's unpaired t test, drug treated groups were compared to vehicle treated groups; n.s.=not significant**

*****=p<0.001; n=3-4**

**Figure 5**

**THE TIME DEPENDENCE OF THE BRONCHOLITIC EFFECT OF DROTAVERINE HCl AND CYCLAMATE SALTS ON ALLEGEN EVOKED BRONCHOCONSTRICTION IN GUINEA PIG AFTER ORAL ADMINISTRATION**

**(Konzett-Rössler method)**

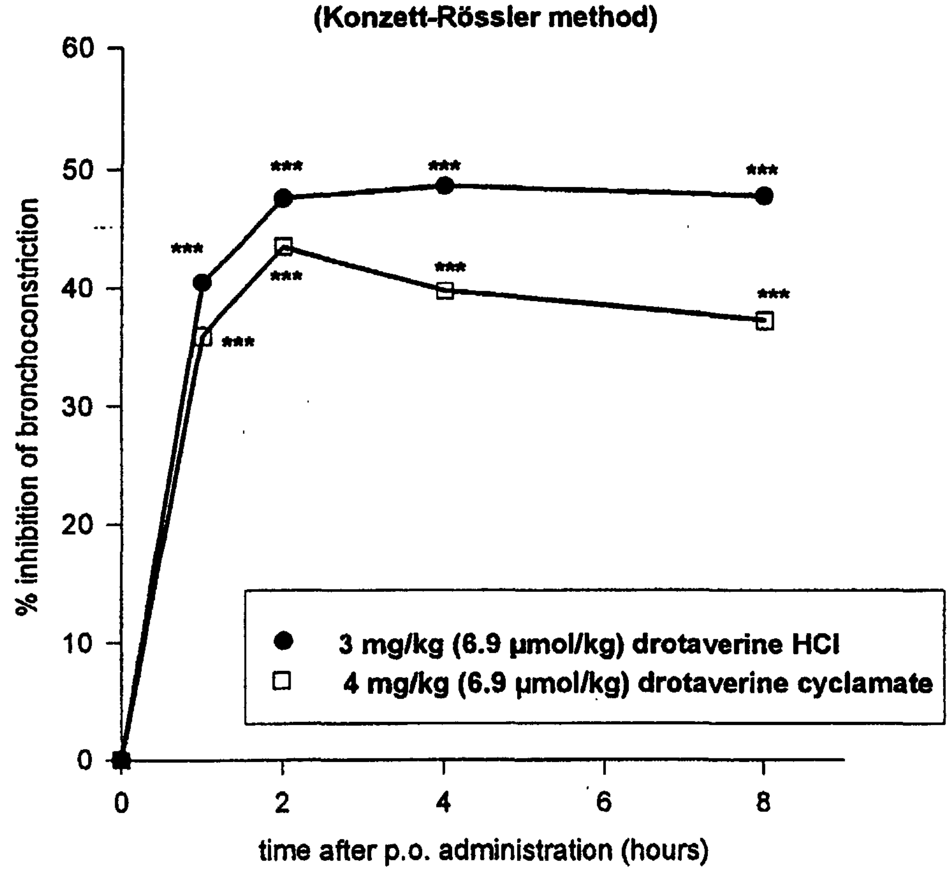


**p.o. treatment in conscious state; statistical analysis by Student's unpaired t test**
**test compound treated groups were compared to vehicle treated groups**
*****=p<0.001; n=4/time point**

**Figure 6**

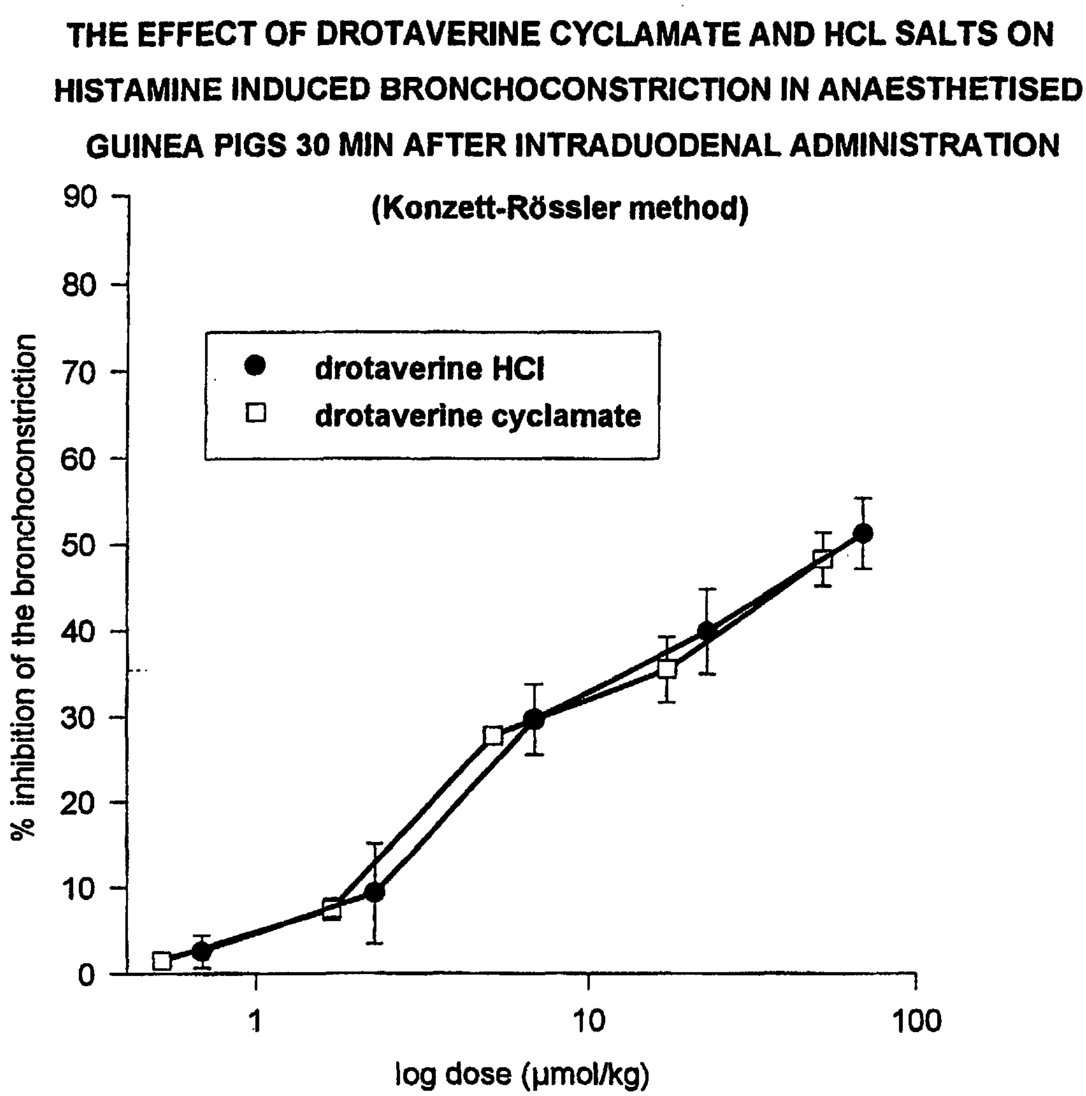


mean±SD; n=3-4

## Figure 7

**THE EFFECT OF SEVEN DAYS DROTAVERINE HCl OR CYCLAMATE SALT ORAL TREATMENT ON ALLERGEN INDUCED BRONCHOCONSTRICTION IN GUINEA PIGS 24 HOURS AFTER THE LAST TREATMENT AND ON THE ACUTE BRONCHOLITIC EFFECT OF THE TWO SALTS**

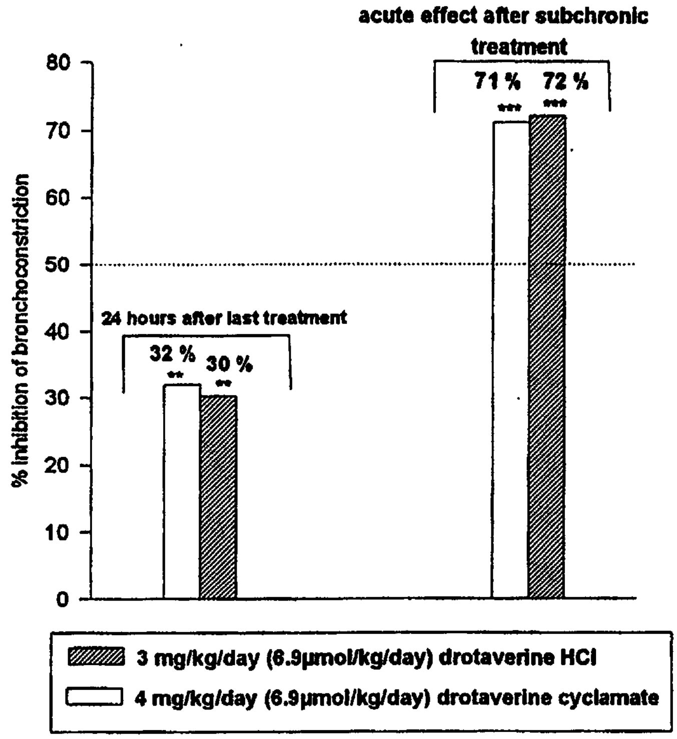


Statistical analysis by Student's unpaired t test; drug treated groups were compared to vehicle treated groups; n=5; **$p<0.01$;***$p<0.001$
single dose/day treatment